# FASCICULE DE BREVET EUROPEEN

(11) **EP 1 712 140 B1**
(45) Date de publication et mention de la délivrance du brevet: **20.08.2008**
(21) Numéro de dépôt: 06290410.7
(22) Date de dépôt: 14.03.2006
(51) Int. Cl.: A23L 1/303, A23L 1/304, A23L 1/305, A23L 1/29, A23L 1/302, A61K 36/258, A61K 35/02

(54) **Composition alimentaire pour prévenir le syndrome de fragilité chez les personnes agées**
Lebensmittelzusammensetzung zur Vorbeugung des Fragilitätssyndrom bei Senioren
Food product for prevention of fragility syndrome with elderly people

(30) Priorité: 14.03.2005 FR 0502488
(43) Date de publication de la demande: 18.10.2006
(73) Titulaire: Larena, 75007 Paris (FR)
(72) Inventeur: Leclerc, Christian, F-18130 Dun Sur Auron (FR)
(74) Mandataire: Breese, Pierre

(56) Documents cités:
- WO-A-02/102163
- WO-A-20/04028550
- WO-A-20/04100896
- US-A- 5 904 924
- US-A- 5 976 548
- US-A- 6 077 828

## Description

La présente invention concerne une composition alimentaire supplétive destinée à être administrée par voie orale au titre de produit diététique. Cette composition vise à prévenir et/ou limiter le syndrome de fragilité chez les personnes âgées.

US 5976548 et WO 2004100896 décrivent des compositions alimentaires supplétives qui sont utilisés pour prévenir les syndrome relatées au veillissement.

Le syndrome de fragilité regroupe des personnes vulnérables dans un état d'équilibre précaire qui sont incapables de répondre de façon adaptée à un stress (pathologique, psychologique ou social). Cet état de fragilité représente un vrai problème de santé public car ces personnes sont celles qui sont les plus consommatrices de soins, les plus à risques de chutes, d'infections, d'hospitalisation et de dépendance. La fragilité est un syndrome clinique qui se développe sous l'action conjuguée du vieillissement physiologique, des habitudes de vie, des changements, des déterminants socioéconomiques, et des pathologies liées à l'âge (les maladies cardiovasculaires, les accidents vasculaires cérébraux ou les cancers...) qui vont conduire tôt ou tard le sujet âgé à la perte de son autonomie. Elle témoigne d'une réduction des réserves physiologiques et d'un amenuisement de l'efficacité des systèmes assurant l'homéostasie du milieu intérieur, limitant ainsi les capacités d'adaptation au stress ou au changement d'environnement. Elle expose à des décompensations fonctionnelles en cascade et à un état morbide incapacitant.

Le syndrome de fragilité, c'est-à-dire le risque de perte d'autonomie, peut se manifester sur le plan clinique par des difficultés à réaliser les activités de la vie quotidienne, une faiblesse, une fatigue générale, une perte de poids et des apports alimentaires insuffisants. Sont associés généralement à ce syndrome : une fonte de la masse musculaire ou sarcopénie, des troubles du maintien de la posture et de la marche, une diminution de la masse osseuse des troubles de la capacité cardio-pulmonaire, un risque de déficit nutritionnel, des infections chroniques.

La sarcopénie correspond à une perte involontaire de masse musculaire et de force musculaire chez une personne âgée bien portante. Chez l'homme, la fonte musculaire se manifeste à partir de 30 ans. Cependant le diagnostic de sarcopénie est difficile car cette baisse de masse musculaire est concomitante à une augmentation de la masse grasse expliquant la stabilité apparente de la masse corporelle.

La perte de masse musculaire correspond à une diminution du nombre total de fibres musculaires et plus particulièrement des fibres de type II. Ainsi au niveau du vastus lateralis (muscle de la cuisse), il existe une diminution de l'ordre de 50 % du nombre de fibres totales entre 20 et 70 ans, diminution qui semble affecter surtout les fibres de type IIb.

Ces modifications de la taille des muscles et de leur composition entraîneraient une diminution de la force musculaire. Cette diminution de la force musculaire devient surtout apparente à partir de l'âge de 50 ans. Les données de l'étude Framingham indiquent que 65% des femmes âgées de plus de 65 ans sont incapables de soulever une charge de 4,5 kg. Par ailleurs, il a été également montré que les muscles des membres inférieurs étaient plus affectés que les muscles des membres supérieurs.

Les conséquences cliniques de la sarcopénie sont multiples et non négligeables : faiblesse musculaire, troubles de la marche et de l'équilibre, fatigabilité, risque accrue de chutes et donc de fractures, perte d'autonomie. Ces facteurs sont autant de risques pour la personne âgée d'être précipitée vers la fragilité voir vers la dépendance. Cette perte de masse musculaire est également un élément prédictif de survie en cas de maladies sévères.

Le système immunitaire a pour rôle de défendre l'organisme contre les agressions externes et internes. Ces agressions pénètrent au niveau de surfaces d'échanges ou de lésions dans les barrières naturelles de protection. Les agressions de type biologique sont le fait d'organismes étrangers, pathogènes ou non, tels les microorganismes (bactéries, champignons), virus ou parasites (protozoaires, helminthes). Les agressions internes sont le fait de cellules devenues tumorales ou dont la fonction met directement ou indirectement les composantes normales de l'organisme (le soi) en danger.

Le vieillissement du système immunitaire est caractérisé par l'involution thymique, qui commence à la puberté et qui est pratiquement totale à l'âge mûr. Cette involution se traduit par une réduction de la taille du thymus et un remplacement progressif de l'espace lymphocytaire et vasculaire par du tissu adipeux.

La diminution de la fonction thymique contribue à l'apparition de lymphocytes T immatures et à une émigration plus lente des lymphocytes T avec apparition d'altérations lymphocytaires. La moelle osseuse produit également moins de lymphocytes B2 naïfs, d'où une capacité réduite à induire des réactions T dépendantes de haute affinité et protectrices tandis que la production sérique d'auto-anticorps anti-idiotypique (AAAI) augmente.

Les dysfonctionnements du système immunitaire sont responsables notamment de la baisse de la résistance des personnes âgées face aux infections. Certaines infections sont ainsi plus répandues chez les personnes âgées que chez les jeunes adultes ; la mortalité et la morbidité relatives aux infections sont également plus fortes. À chaque fois que l'organisme doit faire face à une agression (stress psychique ou maladie), les macrophages libèrent des cytokines pour activer les défenses de l'organisme. Ces cytokines (IL-1, I1-6, TNFα, IL-10 et IL-12) stimulent à la fois le système immunitaire et modifient l'équilibre entre cytokines pro- et anti-inflammatoires. Si la cause de l'agression persiste, les cytokines (IL1, IL6 et TNFα) entraînent une réorganisation importante des métabolismes de l'organisme pour fournir aux cellules activées les nutriments nécessaires. Ainsi, les catabolismes protéiques et lipidiques sont augmentés afin de fournir aux cellules activées les nutriments dont elles ont besoin. Parallèlement, la diminution de fonction des lymphocytes T chez les personnes âgées fragiles conduit les macrophages à sécréter plus de cytokines pour stimuler les lymphocytes T déficients. Une augmentation des taux sériques d'IL-6 est ainsi souvent observée chez les sujets âgés fragiles ou en bonne santé apparente. Le sujet agressé sort donc de toute agression avec des réserves nutritionnelles amoindries, particulièrement chez le sujet âgé où il existe déjà des déséquilibres métaboliques liés au vieillissement (diminution des réserves protéiques musculaires). Les réserves protéiques doivent être rapidement mobilisées en cas d'agression pour fournir des acides aminés et notamment de la glutamine au système immunitaire, au foie, à l'intestin et aux autres organes. On comprend donc aisément qu'une diminution des réserves protéiques musculaires peut avoir des conséquences importantes en cas d'agression. Au stade initial de l'agression, l'organisme va fournir les acides aminés au prix d'une aggravation de la sarcopénie. Par la suite, les muscles n'étant plus capable de fournir assez de carburant des répercussions vont avoir lieu notamment sur le système immunitaire.

Une partie des réserves nutritionnelles consommées ne sera d'ailleurs jamais reconstruite. Ainsi, agression après agression, le sujet âgé consomme progressivement ses réserves et devient de plus en plus fragile par dénutrition progressive. Les déficiences nutritionnelles ou apports insuffisants pourraient faire basculer les personnes âgées en bonne santé vers un état de fragilité et/ou les personnes fragiles vers un état de dépendance. Les protéines de stress ou « heat-shock proteins » (HSP) sont une famille de protéines exerçant de nombreuses fonctions non seulement dans la maturation et le transport des protéines (rôle de chaperonnes moléculaires), mais aussi dans l'activation lymphocytaire et la présentation antigénique.

Leur synthèse est augmentée suite à un stress comme une ischémie, un stress oxydatif, des forces de cisaillement, des inhibiteurs du métabolisme énergétique ou au cours d'infections virales. Elles représentent un moyen de défense de la cellule en réponse à une agression d'où le nom plus approprié de « protéine de stress ». Le vieillissement s'accompagne d'une diminution de la réponse au stress, qui résulte principalement d'une diminution de la capacité des cellules à produire des HSP actives.

On ne sait pas encore très bien si la diminution de la réponse au stress est la cause ou la conséquence du vieillissement. Cette diminution contribue toutefois au renforcement des altérations dues à l'âge, puisque les HSP régulent le renouvellement des protéines, la transduction de signaux, l'expression de gènes et la mort cellulaire. La diminution de la réponse au stress peut aussi favoriser l'accumulation de protéines endommagées, comme les protéines oxydées ou glyquées. L'âge s'accompagnant d'une baisse de production d'ubiquitine, ces protéines ne seront pas éliminées efficacement et continueront de s'accumuler, jusqu'à devenir de puissants inhibiteurs du protéasome. Par « effet boomerang », l'accumulation des protéines endommagées sera renforcée. L'accumulation de protéines oxydées et agrégées pourrait aussi être à l'origine d'une augmentation de l'expression constitutive de certaines HSP.

Le ralentissement de la perte de masse musculaire et du déclin du système immunitaire repose sur le maintien d'un apport protéique quantitatif adéquat. Du fait de leurs particularités métaboliques, la prise en charge nutritionnelle des sujets fragiles doit aussi tenir compte de leur état de stress. En France, les ANC nécessaires pour couvrir la quasi-totalité de la population de sujet âgé considérée sont compris entre 0,9 et 1,1 g/kg/j. En revanche, en situation d'agressions ou de dénutrition, les besoins protéiques de la personne âgée devraient être plus élevés du fait essentiellement de la diminution de la réserve protéique musculaire facilement et rapidement mobilisable. Ces besoins peuvent être couverts par un apport de 15 à 20 g de protéines. Par ailleurs, le ralentissement de la sarcopénie repose sur le maintien d'un apport protéique qualitativement adéquat notamment un apport en protéines riches en acides aminés branchés comme le lactosérum. En effet, chez la personne âgée contrairement à un sujet jeune, un apport en lactosérum résulte en un meilleur gain protéique qu'un apport en caséine. Les acides aminés à chaînes ramifiés (AACR) apparaissent comme un support nutritionnel de la personne âgée intéressant car ces acides aminés et notamment la leucine stimulent de façon significative l'anabolisme musculaire.

En situation de stress, un apport en protéine végétale riche en glutamine et en arginine est recommandé. En effet, dans des conditions d'hypercatabolisme, la demande en glutamine excède les capacités de stockage du muscle squelettique. La glutamine devient dans ce cas un acide aminé essentiel. L'apport de glutamine a deux effets bénéfiques : d'une part la glutamine protège la muqueuse intestinale, d'autre part la glutamine maintient les défenses immunitaires ce qui pourrait contribuer à diminuer les infections. À la suite de stress, une augmentation des besoins en arginine est également observée. Les effets bénéfiques de l'arginine se traduisent par une activation du système immunitaire, une stimulation de la cicatrisation et une amélioration du bilan azoté.

Le ralentissement de la perte de masse musculaire et du déclin du système immunitaire repose également sur le maintien d'un apport en vitamines et minéraux adéquats. En effet, les principaux déficits en vitamines et minéraux chez les personnes âgées sont souvent associés à une carence protéique. C'est le cas en particulier des vitamines du groupe B (B1, B6, B12), de la vitamine D, et de certains minéraux (Zn, Se). D'autre part, un déficit en plusieurs nutriments tels que les vitamines (A, B6, B12, C et E acide folique) et en minéraux (Zinc, sélénium) peut altérer la fonction immunitaire. Cela est lié à l'implication de ces nutriments dans la fonction immunitaire. Chez l'homme les sujets carencés en ces nutriments présentent tous des déficits immunitaires se traduisant par une augmentation des pathologies infectieuses. Des déficiences nutritionnelles ou apports insuffisants pourraient faire basculer les personnes âgées en bonne santé vers un état de fragilité et/ou les personnes fragiles vers un état de dépendance.

Les données actuelles montrent qu'un apport de doses nutritionnelles de vitamines et minéraux et de protéines permet :
- de normaliser les taux circulants de vitamines et minéraux chez les personnes déficientes,
- d'améliorer le statut immunitaire des personnes âgées : augmentation du pourcentage de cellules T CD4+, de l'activité cytotoxique des cellules NK, de la réponse anticorps à un vaccin influenza et une diminution du nombre d'infections,
- d'améliorer l'état et le devenir de patients hospitalisés avec fracture récente : une réduction significative des complications post-fractures, réduction de la durée d'hospitalisation,
- et de restaurer un statut en antioxydant normal chez le sujet âgé présentant des déficits.

En dehors des nutriments essentiels bien identifiés, il existe d'autres composants dont on sait peu de choses mais qui n'en ont pas moins des effets biologiques. Le raisin contient une grande variété de polyphénols (des flavanols, des acides phénoliques, des flavonols et du resvératrol). Ces molécules sont des puissants antioxydants qui préviendraient le développement de maladies chroniques liées à l'âge comme les cancers ou les maladies cardiovasculaires.

Un extrait de *Porphyra umbilicalis* (Porphyral HSP® ) est connu comme ayant des capacités de moduler la synthèse de certaines «Heat shock protein» (HSP) (HSP27, 70, 60). Ces protéines participent au contrôle des infections par trois mécanismes : 1) un rôle immunogène du fait de leur capacité à s'associer aux protéines, 2) un rôle de présentation directe des antigènes à la surface des cellules présentatrices ou encore 3) un rôle de molécule accessoire nécessaire à l'assemblage et/ou la stabilisation du complexe peptide-molécule du complexe majeur d'histocompatibilité (CMH). HSP27 et HSP70 peuvent aussi interférer avec le programme d'entrée en apoptose des cellules endommagées, qui joue un rôle important dans la résolution des agressions cellulaires.

Dans des conditions d'hypercatabolisme, la demande en glutamine excède les capacités de stockage du muscle squelettique. La glutamine devient dans ce cas un acide aminé essentiel.

La glutamine est l'acide aminé le plus abondant dans le sang et le pool des acides aminés libres de l'organisme. Les concentrations intramusculaires et plasmatiques de glutamine diminuent au cours des états d'agression. Beaucoup d'études cliniques réalisées chez les patients agressés ont évalué les avantages cliniques d'un apport en glutamine. Chez l'homme hospitalisé, l'administration de glutamine réduit la fréquence des infections et la durée d'hospitalisation. Chez des patients de soins intensifs, la glutamine augmente le nombre de lymphocytes circulants et leur réponse à une stimulation par des mitogènes.

L'apport de glutamine a deux effets bénéfiques qui pourraient contribuer à diminuer les infections : protection de la muqueuse intestinale et maintient des défenses immunitaires. Les cellules immunitaires activées utilisent préférentiellement la glutamine comme « carburant », au détriment de l'oxydation du glucose. La glutamine régule la prolifération, la différenciation des lymphocytes et stimule la phagocytose des macrophages.

Chez l'homme, dans les conditions normales les besoins en arginine sont couverts par l'apport nutritionnel. Cependant, dans certaines circonstances à la suite de stress (traumatisme) une augmentation des besoins en arginine a été observée. Il est impliqué dans les synthèses des protéines, de l'urée et nucléotides et dans la production d'ATP. C'est aussi le précurseur de l'oxyde nitrique, puissant immunorégulateur cytotoxique pour les cellules tumorales et pour certains micro-organismes. Chez l'homme sain, un apport en arginine stimule la prolifération des lymphocytes sanguins. Elle améliore également la cicatrisation des plaies.

Les effets bénéfiques de l'arginine en phase post-traumatique et infectieuse se traduisent par une activation du système immunitaire, une stimulation de la cicatrisation et une amélioration du bilan azoté.

Les mécanismes d'action de l'arginine sont probablement multiples :
- augmentation de la sécrétion de l'hormone de croissance et de l'insuline qui sont des hormones anabolisantes pour le muscle, et qui stimulent aussi le système immunitaire ;
- augmentation de la production de NO qui est impliqué dans les mécanismes de défense immunitaire ; et
- augmentation de la production de polyamines qui sont importantes dans le contrôle de la division et différenciation cellulaires.

Les acides aminés à chaîne ramifiée (AACR) apparaissent comme un support nutritionnel intéressant chez les sujets fragiles. Une augmentation des apports en leucine pourrait être bénéfique pour stimuler de façon significative l'anabolisme musculaire et donc maintenir la masse protéique musculaire au cours du vieillissement. La leucine favoriserait également l'incorporation des autres acides aminés essentiels.

Les vitamines du groupe B et les vitamines C et E interviennent à la fois sur l'immunité cellulaire et humorale. La vitamine D intervient sur la différenciation cellulaire et l'activité des macrophages. Elle est également citée dans le fonctionnement musculaire où elle pourrait jouer un rôle très important. La vitamine D et ses métabolites pourraient influencer le métabolisme musculaire selon plusieurs voies :
- en modulant la transcription génique ;
- par un effet rapide non-génique par l'intermédiaire de seconds messagers accentuant l'entrée du calcium (Ca) au niveau intracellulaire ; et
- par l'intermédiaire de la modulation du niveau de parathormone (PTH) : une augmentation du niveau de PTH alterne la production et l'utilisation énergétique des muscles et influence le métabolisme des acides aminés et des protéines chez le rat. De plus, la PTH stimule la production de l'interleukine 6 (IL-6), des taux élevés d'IL-6 étant supposés favoriser la sarcopénie. Enfin, le polymorphisme du récepteur à la vitamine D peut expliquer certaines différences de force musculaire entre les individus.

Le magnésium intervient de façon prépondérante sur l'immunité non spécifique, notamment sur l'activité des macrophages. Le zinc est un élément indispensable pour un bon fonctionnement du système immunitaire et la production de cytokines. La carence en zinc entraîne une atrophie thymique, une diminution du nombre de lymphocytes et de cellules NK (Natural Killer). Le zinc joue un rôle dans les processus de réparation et de cicatrisation tissulaires car il favorise la prolifération-cellulaire.

De nombreuses études suggèrent qu'une carence en sélénium (Se) s'accompagne d'une forte diminution de l'immnunocompétence. Le mécanisme d'action paraît étroitement lié à l'aptitude du Se à stimuler l'expression des récepteurs à l'IL-2. Le sélénium intervient dans le métabolisme thyroïdien. La désiodase de type I catalyse la désiodation des hormones thyroïdiennes : desiodation de la tétra-iodothyronine (thyroxine ou T₄) en tri-iodothyronine (T₃). La sélénoprotéine W serait nécessaire à la fonction musculaire. Plusieurs études ont également montré qu'un faible statut sélénique est associé à une incidence significativement plus élevé des épisodes dépressifs et d'autres troubles tels que l'anxiété, la confusion et l'hostilité. Enfin, le sélénium intervient dans la détoxification des métaux lourds, qui peuvent s'accumuler au cours du vieillissement, et exerce un effet activateur de l'élimination des xénobiotiques organiques.

Le sélénium est présent en grandes concentrations dans le foie, la rate et les ganglions lymphatiques. Chez l'homme, son déficit est responsable d'une diminution de la concentration des IgG et IgM circulantes. I1 existerait également une relation entre le déficit en sélénium et la susceptibilité aux infections virales.

La vitamine E est l'anti-oxydant liposoluble majeur de l'organisme. Elle est indispensable au bon fonctionnement et éventuellement à l'amélioration de la réponse immunitaire. Un apport en vitamine E chez les personnes âgées stimule les fonctions immunitaires en augmentant la prolifération des lymphocytes, la production d'anticorps et la réponse d'hypersensibilité retardée (HSR).

La vitamine C est une vitamine hydrosoluble anti-oxydante. Elle est présente en forte concentration dans les leucocytes circulants et est consommée au cours des épisodes infectieux.

Outre son activité provitamine A, d'autres propriétés du β-carotène ont été mises en évidence. Le β-carotène fait partie comme la vitamine C et la vitamine E des systèmes de défenses antiradicalaires non-enzymatiques. I1 réagit de deux façons : par inhibition de la propagation des réactions radicalaires et par neutralisation de l'oxygène singulet.

L'action des caroténoïdes dans l'immunité a été étudiée in vitro et in vivo essentiellement avec le β-carotène. Ce dernier est impliqué dans la prolifération des cellules T et B, il augmente le nombre et la cytotoxicité des cellules NK (Natural Killer), il agirait sur les sécrétions des cytokines et modulerait les défenses non spécifiques.

L'acide folique (vitamine B9) et la vitamine B12 stimulent la prolifération lymphocytaire et la production d'anticorps. Le déficit en vitamine B6 entraîne une diminution du pourcentage et du nombre de lymphocytes circulants, une diminution de la prolifération des cellules T et B lors d'une stimulation par les mitogènes et une réduction de la production de l'interleukine 2 (IL-2). Ces altérations sont partiellement réversibles par réplétion en vitamine B6.

Le zinc stimule l'activité des cellules NK, la prolifération lymphocytaire, la production d'IL-2, la réponse Th1, et la réponse d'HSR.

Les acides aminés soufrés sont essentiels chez l'homme. Les déficits en méthionine et en cystéine entraînent une atrophie du thymus, des ganglions lymphoïdes et de la rate et empêchent la correction de la malnutrition protéino-énergétique. Quand ce déficit est associé à un déficit en isoleucine et en valine, acides aminés également essentiels, il induit une déplétion sévère du tissu lymphoïde intestinal.

L'invention vise à fournir un produit diététique permettant une prise en charge micronutritionnelle en vue de prévenir l'apparition des symptômes liés au syndrome de fragilité chez les personnes âgées et ou de limiter le syndrome de fragilité.

Ainsi, la présente invention a pour premier objet une composition selon la revendication 1, de préférence une composition alimentaire supplétive, comprenant au moins :
(i) des protéines naturelles riches en acides aminés à chaînes ramifiées,
(ii) un acide aminé choisi parmi l'arginine et la glutamine,
(iii) des ginsénosides,
(iv) du zinc,
(v) du sélénium,
(vi) une vitamine choisie parmi les vitamines B1, B2, B3, B5, B6, B8, B9, B12, C, D et E, et
(vii) des caroténoïdes.

De manière surprenante, les inventeurs ont constaté que les composés entrant dans la composition selon l'invention, présentent des modes d'action complémentaires offrant ainsi une synergie pour une action plus efficace.

Par acides aminés à chaînes ramifiées, on entend les trois acides aminés choisis dans le groupe consistant en la valine, la leucine et l'isoleucine.

Les protéines naturelles riches en acides aminés à chaînes ramifiées utilisées dans la composition selon l'invention, sont choisis dans le groupe comprenant les protéines du lactosérum, les protéines de blé, les protéines de riz, les protéines de pois, les protéines de lupin, les protéines de soja ou encore les protéines de caséine. Préférentiellement selon l'invention, on utilise les protéines de lactosérum. Les protéines de lactosérum peuvent se présenter selon l'invention sous la forme de lactosérum total ou de protéines de lactosérum.

Les protéines naturelles riches en acides aminés à chaînes ramifiées peuvent être présentes dans la composition supplétive selon l'invention en une quantité allant de 5 g à 35 g lorsque ladite composition est destinée à une administration journalière, préférentiellement de 10 g à 15 g.

Selon une forme particulière de l'invention, lorsque les protéines du lactosérum sont utilisées, celles-ci peuvent être présentes dans la composition supplétive selon l'invention en une quantité allant de 8 g à 20 g lorsque ladite composition est destinée à une administration journalière, préférentiellement de 10 g à 15 g.

Les acides aminés comme l'arginine et la glutamine peuvent être sous forme libre ou sous la forme de protéines riches en arginine et en glutamine. Ils peuvent provenir d'extrait de céréales ou de légumineuses tels que le riz, le pois, le soja, le blé ou encore le lupin. Préférentiellement selon l'invention, on utilise un extrait de pois.

Lesdits acides aminés, lorsqu'ils sont sous la forme libre peuvent être présents dans la composition en une quantité allant de 3 g à 15 g lorsque ladite composition est destinée à une administration journalière, préférentiellement de 5 g à 9 g.

Lorsqu'ils sont sous la forme de protéines riches en arginine et en glutamine, de préférence de protéines de pois, celles-ci peuvent être présentes dans la composition en une quantité allant de 3 g à 15 g lorsque ladite composition est destinée à une administration journalière, préférentiellement de 5 g à 9 g.

Les ginsénosides utilisés dans la composition selon l'invention, peuvent provenir du ginseng, préférentiellement sous la forme de poudre de ginseng. Lesdits ginsénosides peuvent être présents dans la composition en quantité allant de 1 mg à 10 mg lorsque ladite composition est destinée à une administration journalière, préférentiellement de 2 mg à 4 mg.

Le zinc peut se présenter dans la composition selon l'invention sous la forme de sels de zinc biodisponibles , comme par exemple sous forme d'acétate, de chlorure, de citrate, de gluconate, de lactate, d'oxyde, de carbonate, ou encore de sulfate. Préférentiellement on utilise du sulfate de zinc. Le zinc peut être présent dans la composition en une quantité allant de 10 mg à 20 mg lorsque ladite composition est destinée à une administration journalière, préférentiellement de 13 mg à 17 mg.

Le sélénium peut se présenter dans la composition selon l'invention sous forme de sélénate de sodium, hydrogénosélénite de sodium, sélénite de sodium. Préférentiellement on utilise du sélénite de sodium. Le sélénium peut être présent dans la composition en une quantité allant de 10 µg à 500µg lorsque ladite composition est destinée à une administration journalière, préférentiellement de 25 µg à 100 µg.

Selon une autre forme particulière de l'invention, la composition comprend au moins de la vitamine C, de la vitamine D, de la vitamine E et une vitamine du groupe B choisi parmi les vitamines B1 B2, B3, B5, B6, B8, B9 et B12. De préférence, la composition selon l'invention comprend les vitamines B1, B2, B3, B5, B6, B8, B9, B12, C, D et E.

La vitamine B1 peut se présenter dans la composition selon l'invention sous la forme de chlorhydrate de thiamine, ou encore de mononitrate de thiamine. Préférentiellement on utilise du chlorhydrate de thiamine. La vitamine B1 peut être présente dans la composition en une quantité allant de 0,5 mg à 5 mg lorsque ladite composition est destinée à une administration journalière, préférentiellement de 1 mg à 2 mg.

La vitamine B2 peut se présenter dans la composition selon l'invention sous la forme de riboflavine, ou encore de riboflavine-5'-phosphate de sodium. Préférentiellement on utilise de la riboflavine. La vitamine B2 peut être présente dans la composition selon l'invention en une quantité allant de 0,5 mg à 3 mg lorsque ladite composition est destinée à une administration journalière, préférentiellement de 1 mg à 2 mg.

La vitamine B3 peut se présenter dans la composition sous la forme d'acide nicotinique, ou encore de nicotinamide. Préférentiellement on utilise du nicotinamide. La vitamine B3 peut être présente dans la composition selon l'invention en une quantité allant de 5 mg à 30 mg lorsque ladite composition est destinée à une administration journalière, préférentiellement de 15 mg à 20 mg.

La vitamine B5 peut se présenter dans la composition selon l'invention sous la forme de D-pantothénate de calcium, de D-panthoténate de sodium, ou encore de dexpantoténol. Préférentiellement, on utilise de la D-pantothénate de calcium. La vitamine B5 peut être présente dans la composition selon l'invention en une quantité allant de 1 mg à 10 mg lorsque ladite composition est destinée à une administration journalière, préférentiellement de 5 mg à 8 mg.

La vitamine B6 peut se présenter dans la composition selon l'invention sous la forme de chlorhydrate de pyridoxine, de pyridoxine-5'-phosphate, ou encore de dipalmitate de pyridoxine. Préférentiellement on utilise du chlorhydrate de pyridoxine. La vitamine B6 peut être présente dans la composition en une quantité allant de 0,5 mg à 5 mg lorsque ladite composition est destinée à une administration journalière, préférentiellement de 1 mg à 3 mg.

La vitamine B8 peut se présenter dans la composition selon l'invention sous la forme de D-biotine. Préférentiellement on utilise selon l'invention de la D-biotine. La vitamine B8 peut être présente dans la composition en une quantité allant de 0,1 mg à 1 mg lorsque ladite composition est destinée à une administration journalière, préférentiellement de 0,1 mg à 0,3 mg.

La vitamine B9 peut se présenter dans la composition selon l'invention sous la forme d'acide ptéroylmonoglutamique. Préférentiellement on utilise selon l'invention de l'acide ptéroylmonoglutamique. La vitamine B9 peut être présente dans la composition en une quantité allant de 0,1 mg à 0,5 mg lorsque ladite composition est destinée à une administration journalière, préférentiellement de 0,15 mg à 0,3 mg.

La vitamine B12 peut se présenter dans la composition selon l'invention sous la forme de cyanocobalamine, ou encore d'hydroxocobalamine. Préférentiellement on utilise de la cyanocobalamine. La vitamine B12 peut être présente dans la composition en une quantité allant de 0,1 µg à 3 µg, lorsque ladite composition est destinée à une administration journalière préférentiellement de 0,5 µg à 1,5 µg.

La vitamine D peut se présenter dans la composition sous une forme très biodisponible telle que du cholécalciférol (D₃), ou encore de l'ergocalciférol (D₂). Préférentiellement selon l'invention, on utilise du cholécalciférol. La vitamine D peut être présente dans la composition en une quantité allant de 1 µg à 20 µg lorsque ladite composition est destinée à une administration journalière, préférentiellement de 5 µg à 20 µg.

La vitamine C peut se présenter dans la composition sous une forme très biodisponible de vitamine C comme par exemple un extrait végétal riche en vitamine C comme un d'extrait d'acérola, de l'acide L-ascorbique, du L-ascorbate de sodium, du L-ascorbate de calcium, du L-ascorbate de potassium ou du L-ascorbyl 6-palmitate. Préférentiellement on utilise un extrait végétal riche en vitamine C, très préférentiellement un extrait d'acérola. La vitamine C peut être présent dans la composition en une quantité allant de 40 mg à 100 mg lorsque ladite composition est destinée à une administration journalière, préférentiellement de à 50 mg à 80 mg.

La vitamine E peut se présenter dans la composition sous la forme de D-α-tocophérol, de DL-α-tocophérol, d'acétate de D-α-tocophérol, d'acétate de DL-α-tocophérol ou de succinate de D-α-tocophérol. Préférentiellement on utilise de l'acétate de DL-α-tocophérol. La vitamine E peut être présente dans la composition en une quantité allant de 1 mg à 25 mg lorsque ladite composition est destinée à une administration journalière, préférentiellement de 8 mg à 12 mg.

Les caroténoïdes utilisés dans la composition selon l'invention, peuvent être par exemple du β-carotène, du lycopène, de la lutéine, de l'asthaxanthine, de la zéaxanthine ou encore de la canthaxanthine. Préférentiellement, on utilise du β-carotène. Dans la composition selon l'invention, le caroténoïde peut être présent en une quantité allant de 1 mg à 20 mg lorsque ladite composition est destinée à une administration journalière, préférentiellement de 4,5 mg à 20 mg.

Selon un mode de réalisation préféré, la composition selon l'invention comprend également un extrait d'algue.

L'extrait d'algue peut être tout extrait d'algue connu pour avoir la capacité d'augmenter les expressions des HSP. Particulièrement, l'extrait d'algue peut être un extrait de *Porphyra umbilicalis.* Selon l'invention, l'extrait d'algue peut être présent dans la composition en une quantité allant de 1 mg à 750 mg lorsque ladite composition est destinée à une administration journalière, préférentiellement de 10 mg à 500 mg.

Sous une forme particulière de l'invention, l'extrait de *Porphyra umbilicalis* est présent dans la composition sous la forme de Porphyral HSP®. Sous cette forme particulière de l'invention, le Porphyral HSP® peut être présent dans la composition en une quantité allant de 1 mg à 500 mg lorsque ladite composition est destinée à une administration journalière, préférentiellement de 50 mg à 200 mg.

Selon un autre mode de réalisation préféré, la composition selon l'invention comprend également des polyphénols.

Les polyphénols peuvent être présents dans la composition sous forme d'extrait total de raisin, de cacao, de pomme, de thé vert, de vin rouge, ou encore de fruits rouges (fraise, myrtille, canneberge). Préférentiellement on utilise un extrait total de raisin. Les polyphénols peuvent être présents dans la composition en une quantité allant de 50 mg à 500 mg lorsque ladite composition est destinée à une administration journalière, préférentiellement de 80 mg à 150 mg.

Selon un autre mode de réalisation préféré, la composition selon l'invention comprend également de la méthionine.

Selon un autre mode de réalisation préféré, la composition selon l'invention comprend également du magnésium.

Le magnésium peut être présent sous forme de d'acétate de magnésium, de carbonate de magnésium, de chlorure de magnésium, de gluconate de magnésium, de citrate de magnéisum, de glycérophosphate de magnésium, de lactate de magnésium, d'hydroxyde de magnésium, d'oxyde de magnésium, de sulfate de magnésium ou d'orthophosphate de magnésium. Préférentiellement, on utilise le carbonate de magnésium. Le magnésium peut être présent dans la composition en une quantité allant de 10 mg à 800 mg lorsque ladite composition est destinée à une administration journalière, préférentiellement de 100 mg à 200 mg.

Selon un autre mode de réalisation préféré, la composition selon l'invention comprend également du manganèse.

Le manganèse peut être présent sous forme de carbonate de manganèse, de chlorure de manganèse, de citrate de manganèse, de gluconate de manganèse, de glycérophosphate de manganèse ou de sulfate de manganèse. Préférentiellement, on utilise le sulfate de manganèse. Le manganèse peut être présent dans la composition en une quantité allant de 0,2 mg à 10 mg lorsque ladite composition est destinée à une administration journalière, préférentiellement de 0,8 mg à 3 mg.

Ladite composition peut en outre comprendre tout autre ingrédient actif. Les compositions selon l'invention, peuvent aussi, selon la formulation choisie, comprendre tout excipient approprié, tel un liant, un lubrifiant, un colorant, un arôme, un suppresseur de goût.

Les compositions selon l'invention peuvent se présenter sous la forme de poudres, de comprimés, de granules, de poudre en gélules, de granules en gélules, huileuses en capsule.

Une composition préférée selon l'invention comprend au moins :
- des protéines de lactosérum, de préférence 11,7 g,
- des protéines de pois, de préférence 7 g,
- des ginsénosides sous forme de poudre de ginseng, de préférence 2,9 mg de ginsénosides sous forme de 29 mg de poudre de ginseng,
- du zinc, de préférence 15 mg,
- du sélénium, de préférence 60 µg,
- de la vitamine B1 de préférence 1,4 mg,
- de la vitamine B2, de préférence 1,6 mg,
- de la vitamine B3, de préférence 18 mg,
- de la vitamine B5, de préférence 6 mg,
- de la vitamine B6, de préférence 2 mg,
- de la vitamine B8, de préférence 0,15 mg,
- de la vitamine B9, de préférence 0,20 mg,
- de la vitamine B12, de préférence 1 µg,
- de la vitamine D, de préférence 5 µg,
- de la vitamine C, de préférence 60 mg,
- de la vitamine E, de préférence 10 mg,
- du Porphyral HSP en tant qu'extrait d'algue, de préférence 100 mg,
- des polyphénols sous forme de poudre de raisin, de préférence 100 mg de polyphénols sous forme de 110 mg de poudre de raisin, et
- du β-carotène, de préférence 4,8 mg.
Ladite composition peut en outre comprendre
- de la méthionine, de préférence 58 mg,
- du magnésium, de préférence 150 mg, et
- du manganèse, de préférence 1,75 mg.

Les quantités correspondent à la prise en une dose de la quantité nécessaire à ingérer par jour. Dans le cas d'une administration différente de la composition selon l'invention, notamment à raison de deux, trois ou quatre fois par jour, l'homme du métier pourra adapter sans difficulté les quantités des différents composés décrites précédemment de sorte d'obtenir la composition selon l'invention adaptée.

Sous une forme particulière de réalisation de l'invention, la composition peut, pour des raisons organoleptiques, comprendre une partie sous forme de poudre et une seconde partie sous forme de comprimé.

Une composition préférée de l'invention comprend une poudre à diluer et un comprimé à prendre simultanément. Ladite poudre à diluer comprend :
- 11,7 g de protéines de lactosérum,
- 7 g de protéines de pois,
- 2,9 mg de ginsénosides sous forme de 29 mg de poudre de ginseng,
- 15 mg de zinc,
- 60 µg de sélénium,
- 1,4 mg de vitamine B1,
- 1,6 mg de vitamine B2,
- 18 mg de vitamine B3,
- 6 mg de vitamine B5,
- 2 mg de vitamine B6,
- 0,15 mg de vitamine B8,
- 0,20 mg de vitamine B9
- 1 µg de vitamine B12,
- 5 µg de vitamine D,
- 60 mg de vitamine C ;
- 10 mg de vitamine E,
- 4,8 mg de β-carotène,
- 58 mg de méthionine,
- 150 mg de magnésium,
- 1,75 mg de manganèse ;
et ledit comprimé comprend .
- 100 mg de Porphyral HSP,
- 100 mg de polyphénols sous forme de 110 mg de poudre de raisin.

Ces quantités correspondent à la prise en une dose de la quantité nécessaire à ingérer par jour.

L'invention a par ailleurs pour objet l'utilisation de la composition selon l'invention, pour la préparation d'une composition destinée à la prévention et/ou au traitement du syndrome de fragilité chez les personnes âgées, de la sarcopénie, à prévenir le déclin du système immunitaire, à prévenir l'immunoscenescence, à prévenir le déclin des réponses immunitaires, à prévenir et/ou à réduire le nombre d'épisodes infectieux, à restaurer un statut anti-oxydant chez les personnes âgées, à prévenir et/ou traiter les conséquences post-traumatiques (ex post-hospitalisation pour fracture), à prévenir les troubles du maintien de la posture et de la marche, à prévenir le déficit protéino-énergétique chez les personnes âgées, à lutter contre la perte de masse musculaire et la diminution de la force musculaire, à la prévention de l'état de vulnérabilité et de fragilité chez la personne âgée.

Ladite composition peut prendre la forme d'une composition alimentaire, d'un complément alimentaire, ou d'un produit diététique.

Avantageusement, la composition selon l'invention est destinée à prévenir et/ou traiter le syndrome de fragilité.

## Revendications

1. Une composition, de préférence une composition alimentaire supplétive, comprenant au moins :
(i) des protéines naturelles riches en acides aminés à chaînes ramifiées choisies dans le groupe comprenant les protéines du lactosérum, les protéines de blé, les protéines de riz, les protéines de pois, les protéines de lupin, les protéines de soja et les protéines de caséine,
(ii) un acide aminé choisi parmi l'arginine et la glutamine,
(iii) des ginsénosides,
(iv) du zinc,
(v) du sélénium,
(vi) une vitamine choisie parmi les vitamines B1, B2, B3, B5, B6, B8, B9, B12, C, D et E, et
(vii) des caroténoïdes.

2. La composition selon la revendication 1, **caractérisée en ce que caractérisée en ce que** l'arginine et la glutamine sont sous forme libre ou sous la forme de protéines riches en arginine et en glutamine choisies dans le groupe comprenant les extraits de céréales et de légumineuses tels que le riz, le pois, le soja, le blé ou encore le lupin, de préférence le pois.

3. La composition selon l'une quelconque des revendications 1 à 2, **caractérisée en ce que** les ginsénosides proviennent du ginseng, de préférence lesdits ginsénosides sont sous la forme de poudre de ginseng.

4. La composition selon l'une quelconque des revendications 1 à 3, **caractérisée en ce que** le zinc est sous la forme de sels de zinc biodisponibles, comme par exemple sous forme d'acétate, de chlorure, de citrate, de gluconate, de lactate, d'oxyde, de carbonate, ou encore de sulfate, de préférence sous la forme de sulfate de zinc.

5. La composition selon l'une quelconque des revendications 1 à 4, le sélénium est sous la forme de sélénate de sodium, d'hydrogénosélénite de sodium ou de sélénite de sodium, de préférence de sélénite de sodium.

6. La composition selon l'une quelconque des revendications 1 à 5, **caractérisée en ce que** les caroténoïdes sont choisis dans le groupe comprenant le β-carotène, le lycopène, la lutéine, l'asthaxanthine, la zéaxanthine et la canthaxanthine, de préférence le β-carotène.

7. La composition selon l'une quelconque des revendications 1 à 6, **caractérisée en ce qu'**elle comprend au moins de la vitamine C, de la vitamine D, de la vitamine E et au moins une vitamine du groupe B choisie dans le groupe comprenant les vitamines B1, B2, B3, B5, B6, B8, B9 et B12, de préférence elle comprend les vitamines B1, B2, B3, B5, B6, B8, B9, B12, C, D et E.

8. La composition selon la revendication 7, **caractérisée en ce que** :
(i) la vitamine B1 est sous la forme de chlorhydrate de thiamine ou de mononitrate de thiamine, de préférence sous la forme de chlorhydrate de thiamine ;
(ii) la vitamine B2 est sous la forme de riboflavine ou de riboflavine-5'-phosphate de sodium, de préférence sous la forme de riboflavine ;
(iii) la vitamine B3 est sous la forme d'acide nicotinique ou de nicotinamide, de préférence sous la forme de nicotinamide ;
(iv) la vitamine B5 est sous la forme de D-pantothénate de calcium, de D-panthoténate de sodium ou de dexpantoténol, de préférence sous la forme de D-pantothénate de calcium ;
(v) la vitamine B6 est sous la forme de chlorhydrate de pyridoxine, de pyridoxine-5'-phosphate ou de dipalmitate de pyridoxine, de préférence sous la forme de chlorhydrate de pyridoxine ;
(vi) la vitamine B8 est sous la forme de D-biotine ;
(vii) la vitamine B9 est sous la forme d'acide ptéroylmonoglutamique ;
(viii) la vitamine B12 est sous la forme de cyanocobalamine ou d'hydroxocobalamine, de préférence sous la forme de cyanocobalamine ;
(ix) la vitamine C est sous la forme d'acide L-ascorbique, de L-ascorbate de sodium, de L-ascorbate de calcium, de L-ascorbate de potassium, de L-ascorbyl 6-palmitate ou d'un extrait végétal riche en vitamine C comme un d'extrait d'acérola, de préférence sous la forme d'un extrait végétal riche en vitamine C ;
(x) la vitamine D est sous la forme de cholécalciférol (D₃) ou d'ergocalciférol (D₂), de préférence sous la forme de cholécalciférol (D₃) ; et
(xi) la vitamine E est sous la forme de D-α-tocophérol, de DL-α-tocophérol, d'acétate de D-α-tocophérol, d'acétate de DL-α-tocophérol ou de succinate de D-α-tocophérol, préférentiellement sous la forme d'acétate de DL-α-tocophérol.

9. La composition selon l'une quelconque des revendications 1 à 8, **caractérisée en ce qu'**elle comprend en outre un extrait d'algue capable d'augmenter les effets des HSP, de préférence un extrait de *Porphyra umbilicalis.*

10. La composition selon l'une quelconque des revendications 1 à 9, **caractérisée en ce qu'**elle comprend en outre des polyphénols, lesquels polyphénols sont présents sous forme d'extrait total de raisin, de cacao, de pomme, de thé vert, de vin rouge ou de fruits rouges comme la fraise, la myrtille ou le canneberge, de préférence les polyphénols sont présents sous forme d'extrait total de raisin.

11. La composition selon l'une quelconque des revendications précédentes, **caractérisée en ce que** ladite composition, lorsqu'elle est destinée à une administration journalière comprend :
(i) des protéines naturelles riches en acides aminés à chaînes ramifiées choisies dans le groupe comprenant les protéines de blé, les protéines de riz, les protéines de pois, les protéines de lupin, les protéines de soja et les protéines de caséine, en une quantité allant de 5 g à 35 g, de préférence de 10 g à 15 g ;
(ii) des protéines du lactosérum en une quantité allant de 8 g à 20 g, de préférence de 10 g à 15 g ;
(iii) des protéines riches en arginine et en glutamine en une quantité allant de 3 g à 15 g, de préférence de 5 g à 9 g ;
(iv) des ginsénosides en une quantité allant de 1 mg à 10 mg, de préférence de 2 mg à 4 mg ;
(v) du zinc en une quantité allant de 10 mg à 20 mg, de préférence entre 13 mg et 17 mg ;
(vi) du sélénium en une quantité allant de 10 µg à 500 µg, de préférence de 25 µg à 100 µg ;
(vii) de la vitamine B1 en une quantité allant de 0,5 mg à 5 mg, de préférence de 1 mg à 2 mg ;
(viii) de la vitamine B2 en une quantité allant de 0,5 mg à 3 mg, de préférence de 1 mg à 2 mg ;
(ix) de la vitamine B3 en une quantité allant de 5 mg à 30 mg, de préférence de 15 mg à 20 mg ;
(x) de la vitamine B5 en une quantité allant de 1 mg à 10 mg, de préférence de 5 mg à 8 mg ;
(xi) de la vitamine B6 en une quantité allant de 0,5 mg à 5 mg, de préférence de 1 mg à 3 mg ;
(xii) de la vitamine B8 en une quantité allant de 0,1 mg à 1 mg, de préférence de 0,1 mg à 0,3 mg ;
(xiii) de la vitamine B9 en une quantité allant de 0,1 mg à 0,5 mg, de préférence de 0,15 mg à 0,3 mg ;
(xiv) de la vitamine B12 en une quantité allant de 0,1 µg à 3 µg, de préférence de 0,5 µg à 1,5 µg ;
(xv) de la vitamine C en une quantité allant de 40 mg à 100 mg, de préférence de à 50 mg à 80 mg ;
(xvi) de la vitamine D en une quantité allant de 1 µg à 20 µg, de préférence de 5 µg à 20 µg ;
(xvii) de la vitamine E en une quantité allant de 1 mg à 25 mg, de préférence de 8 mg à 12 mg ;
(xviii) des caroténoïdes en une quantité allant de 1 mg à 20 mg, de préférence de 4,5 mg à 20 mg ;
(xix) un extrait d'algue en une quantité allant de 1 mg à 750 mg, de préférence de 1 mg à 500 mg ; et
(xx) des polyphénols en une quantité allant de 50 mg à 500 mg, de préférence de 80 mg à 150 mg.

12. La composition selon l'une quelconque des revendications précédentes, **caractérisée en ce qu'**elle comprend au moins :
(i) des protéines de lactosérum, de préférence 11,7 g,
(ii) des protéines de pois, de préférence 7 g,
(iii) des ginsénosides sous forme de poudre de ginseng, de préférence 2,9 mg de ginsénosides sous forme de 29 mg de poudre de ginseng,
(iv) du zinc, de préférence 15 mg,
(v) du sélénium, de préférence 60 µg,
(vi) de la vitamine B1, de préférence 1,4 mg,
(vii) de la vitamine B2, de préférence 1,6 mg,
(viii) de la vitamine B3, de préférence 18 mg,
(ix) de la vitamine B5, de préférence 6 mg,
(x) de la vitamine B6, de préférence 2 mg,
(xi) de la vitamine B8, de préférence 0,15 mg,
(xii) de la vitamine B9, de préférence 0,20 mg,
(xiii) de la vitamine B12, de préférence 1 µg,
(xiv) de la vitamine D, de préférence 5 µg,
(xv) de la vitamine C, de préférence 60 mg,
(xvi) de la vitamine E, de préférence 10 mg,
(xvii) du Porphyral HSP en tant qu'extrait d'algue, de préférence 100 mg,
(xviii) des polyphénols sous forme de poudre de raisin, de préférence 100 mg de polyphénols sous forme de 110 mg de poudre de raisin, et
(xix) du β-carotène, de préférence 4,8 mg.

13. La composition selon l'une quelconque des revendications précédentes, **caractérisée en ce qu'**elle comprend en outre :
(i) de la méthionine, de préférence 58 mg,
(ii) du magnésium, de préférence 150 mg, et/ou
(iii) du manganèse, de préférence 1,75 mg.

14. Une utilisation d'une composition selon l'une quelconque des revendications 1 à 13 pour la préparation d'une composition destinée à la prévention et/ou au traitement du syndrome de fragilité chez les personnes âgées, de la sarcopénie, à prévenir le déclin du système immunitaire, à prévenir l'immunoscenescence, à prévenir le déclin des réponses immunitaires cellulaires, à prévenir et/ou à réduire le nombre d'épisodes infectieux, à restaurer un statut anti-oxydant chez les personnes âgées, à prévenir et/ou traiter les conséquences post-traumatiques (ex post-hospitalisation pour fracture), à prévenir les troubles du maintien de la posture et de la marche, à prévenir le déficit protéino-énergétique chez les personnes âgées, à lutter contre la perte de masse musculaire et la diminution de la force musculaire, à la prévention de l'état de vulnérabilité et de fragilité chez la personne âgée.

## Claims

1. Formulation, preferentially a nutritional supplement formulation, comprising at least:
(i) natural proteins rich in branched-chain amino acids selected from the group comprising whey proteins, wheat proteins, rice proteins, pea proteins, lupin proteins, soy proteins and casein proteins,
(ii) an amino acid selected from arginine and glutamine,
(iii) ginsenosides,
(iv) zinc,
(v) selenium,
(vi) a vitamin selected from vitamins B1, B2, B3, B5, B6, B8, B9, B12, C, D and E, and
(vii) carotenoids.

2. Formulation according to claim 1, **characterised in that** arginine and glutamine are in free form or in the form of proteins rich in arginine and glutamine selected from the group comprising cereal and leguminous extracts such as rice, pea, soy, wheat or lupin, preferentially pea.

3. Formulation according to any of claims 1 to 2, **characterised in that** the ginsenosides are obtained from ginseng, preferentially said ginsenosides are in the form of ginseng powder.

4. Formulation according to any of claims 1 to 3, **characterised in that** the zinc is in the form of bioavailable zinc salts, such as for example in acetate, chloride, citrate, gluconate, lactate, oxide, carbonate or sulphate form, preferentially in the form of zinc sulphate.

5. Formulation according to any of claims 1 to 4, **characterised in that** the selenium is in the form of sodium selenate, sodium hydrogen selenite or sodium selenite, preferentially sodium selenite.

6. Formulation according to any of claims 1 to 5, **characterised in that** the carotenoids are selected from the group comprising β-carotene, lycopene, lutein, asthaxanthine, zeaxanthine and canthaxanthine, preferentially β-carotene.

7. Formulation according to any of claims 1 to 6, **characterised in that** it comprises at least vitamin C, vitamin D, vitamin E and at least one B vitamin selected from the group comprising vitamins B1, B2, B3, B5, B6, B8, B9 and B12, preferentially, it comprises vitamins B1, B2, B3, B5, B6, B8, B9, B12, C, D and E.

8. Formulation according to claim 7, **characterised in that**:
(i) the vitamin B1 is in the form of thiamine hydrochloride or thiamine mononitrate, preferentially in the form of thiamine hydrochloride;
(ii) the vitamin B2 is in the form of riboflavin or riboflavin-5'-phosphate sodium, preferentially in the form of riboflavin;
(iii) the vitamin B3 is in the form of nicotinic acid or nicotinamide, preferentially in the form of nicotinamide;
(iv) the vitamin B5 is in the form of calcium D-panthothenate, sodium D-panthothenate or dexpantotenol, preferentially in the form of calcium D-panthothenate;
(v) the vitamin B6 is in the form of pyroxidine hydrochloride, pyridoxine-5'-phosphate or pyridoxine dipalmitate, preferentially in the form of pyridoxine hydrochloride;
(vi) the vitamin B8 is in the form of D-biotin;
(vii) the vitamin B9 is in the form of pteroylmonoglutamic acid;
(viii) the vitamin B12 is in the form of cyanocobalamin or hydroxocobalamin, preferentially in the form of cyanocobalamin;
(ix) the vitamin C is in the form of L-ascorbic acid, sodium L-ascorbate, calcium L-ascorbate, potassium L-ascorbate, L-ascorbyl 6-palmitate or a plant extract rich in vitamin C such as acerola extract, preferentially in the form of a plant extract rich in vitamin C;
(x) the vitamin D is in the form of cholecalciferol (D₃) or ergocalciferol (D₂), preferentially in the form of cholecalciferol (D₃); and
(xi) the vitamin E is in the form of D-α-tocopherol, DL-α-tocopherol, D-α-tocopherol acetate, DL-α-tocopherol acetate, D-α-tocopherol succinate, preferentially in the form of DL-α-tocopherol acetate.

9. Formulation according to any of claims 1 to 8, **characterised in that** it also comprises a seaweed extract capable of increasing the effects of HSPs, preferentially a Porphyra umbilicalis extract.

10. Formulation according to any of claims 1 to 9, **characterised in that** it also comprises polyphenols, which are present in the form of total extract of grape, cocoa, apple, green tea, red wine or berries such as strawberry, blueberry or cranberry, preferentially the polyphenols are present in the form of total extract of grape.

11. Formulation according to any of the above claims, **characterised in that** said formulation, when intended for daily administration, comprises:
(i) natural proteins rich in branched-chain amino acids selected from the group comprising wheat proteins, rice proteins, pea proteins, lupin proteins, soy proteins and casein proteins, in a quantity ranging from 5 g to 35 g, preferentially from 10 g to 15 g;
(ii) whey proteins in a quantity ranging from 8 g to 20 g, preferentially from 10 g to 15 g;
(iii) proteins rich in arginine and glutamine in a quantity ranging from 3 g to 15 g, preferentially from 5 g to 9 g;
(iv) ginsenosides in a quantity ranging from 1 mg to 10 mg, preferentially from 2 mg to 4 mg;
(v) zinc in a quantity ranging from 10 mg to 20 mg, preferentially between 13 mg to 17 mg;
(vi) selenium in a quantity ranging from 10 µg to 500 µg, preferentially from 25 µg to 100 µg;
(vii) vitamin B1 in a quantity ranging from 0.5 mg to 5 mg, preferentially from 1 mg to 2 mg;
(viii) vitamin B2 in a quantity ranging from 0.5 mg to 3 mg, preferentially from 1 mg to 2 mg;
(ix) vitamin B3 in a quantity ranging from 5 mg to 30 mg, preferentially from 15 mg to 20 mg;
(x) vitamin B5 in a quantity ranging from 1 mg to 10 mg, preferentially from 5 mg to 8 mg;
(xi) vitamin B6 in a quantity ranging from 0.5 mg to 5 mg, preferentially from 1 mg to 3 mg;
(xii) vitamin B8 in a quantity ranging from 0.1 mg to 1 mg, preferentially from 0.1 mg to 0.3 mg;
(xiii) vitamin B9 in a quantity ranging from 0.1 mg to 0.5 mg, preferentially from 0.15 mg to 0.3 mg;
(xiv) vitamin B12 in a quantity ranging from 0.1 µg to 3 µg, preferentially from 0.5 µg to 1.5 µg;
(xv) vitamin C in a quantity ranging from 40 mg to 100 mg, preferentially from 50 mg to 80 mg;
(xvi) vitamin D in a quantity ranging from 1 µg to 20 µg, preferentially from 5 µg to 20 µg;
(xvii) vitamin E in a quantity ranging from 1 mg to 25 mg, preferentially from 8 mg to 12 mg;
(xviii) carotenoids in a quantity ranging from 1 mg to 20 mg, preferentially from 4.5 mg to 20 mg;
(xix) seaweed extract in a quantity ranging from 1 mg to 750 mg, preferentially from 1 mg to 500 mg; and
(xx) polyphenols in a quantity ranging from 50 mg to 500 mg, preferentially from 80 mg to 150 mg.

12. Formulation according to any of the above claims, **characterised in that** it comprises at least:
(i) whey proteins, preferentially 11.7 g,
(ii) pea proteins, preferentially 7 g,
(iii) ginsenosides in the form of ginseng powder, preferentially 2.9 mg of ginsenosides in the form of 29 mg of ginseng powder,
(iv) zinc, preferentially 15 mg,
(v) selenium, preferentially 60 µg,
(vi) vitamin B1, preferentially 1.4 mg,
(vii) vitamin B2, preferentially 1.6 mg,
(viii) vitamin B3, preferentially 18 mg,
(ix) vitamin B5, preferentially 6 mg,
(x) vitamin B6, preferentially 2 mg,
(xi) vitamin B8, preferentially 0.15 mg,
(xii) vitamin B9, preferentially 0.20 mg,
(xiii) vitamin B12, preferentially 1 µg,
(xiv) vitamin D, preferentially 5 µg,
(xv) vitamin C, preferentially 60 mg,
(xvi) vitamin E, preferentially 10 mg,
(xvii) Porphyral HSP as a seaweed extract, preferentially 100 mg,
(xviii) polyphenols in the form of grape powder, preferentially 100 mg of polyphenols in the form of 110 mg of grape powder, and
(xix) β-carotene, preferentially 4.8 mg.

13. Formulation according to any of the above claims, **characterised in that** it also comprises:
(i) methionine, preferentially 58 mg,
(ii) magnesium, preferentially 150 mg, and/or
(iii) manganese, preferentially 1.75 mg.

14. Use of a formulation according to any of claims 1 to 13 for the preparation of a formulation intended to prevent and/or treat fragility syndrome in elderly subjects, sarcopenia, prevent immune system decline, prevent immunoscenescence, prevent cellular immune response decline, prevent and/or reduce the number of infectious episodes, restore antioxidant status in elderly subjects, prevent and/or treat post-traumatic effects (e.g. post-hospitalisation for fracture), prevent postural maintenance and walking disorders, prevent protein-energy deficiency in elderly subjects, combat muscle mass loss and reduction in muscle strength, prevent vulnerable and fragile conditions in elderly subjects.

## Patentansprüche

1. Verbindung, vorzugsweise eine Nahrungszusatz-Verbindung umfassend mindestens:
(i) natürliche Proteine, reich an Aminosäuren mit verzweigten Ketten, gewählt aus der Gruppe umfassend Molkenproteine, die Weizenproteine, die Reisproteine, die Erbsenproteine, die Lupinenproteine, die Sojaproteine und die Kaseinproteine,
(ii) eine Aminosäure, gewählt unter dem Arginin und dem Glutamin,
(iii) Ginsenoside,
(iv) Zink,
(v) Selen,
(vi) ein Vitamin, gewählt unter den Vitaminen B1, B2, B3, B5, B6, B8,B9, B12, C, D und E, und
(vii) Carotinoide.

2. Verbindung nach Anspruch 1, **dadurch gekennzeichnet, dass** das Arginin und das Glutamin in freier Form oder in der Form von Vitaminen vorliegen, die reich an Arginin und an Glutamin sind, ausgewählt aus der Gruppe umfassend die Extrakte aus Getreide und Hülsenfrüchten wie z.B. Reis, Erbsen, Soja, Weizen oder auch Lupinen, vorzugsweise Erbsen.

3. Verbindung nach einem der Ansprüche 1 bis 2, **dadurch gekennzeichnet, dass** die Ginsenoside aus Ginseng stammen, vorzugsweise liegen die Ginsenoside in der Form von Ginsengpuder vor.

4. Verbindung nach einem der Ansprüche 1 bis 3, **dadurch gekennzeichnet, dass** das Zink in Form von biologisch verfügbaren Zinksalzen vorliegt, wie z.B. in der Form von Azetat, Chlorid, Zitrat, Glukonat, Laktat, Oxyd, Karbonat oder auch Sulfat, vorzugsweise in der Form von Zinksulfat.

5. Verbindung nach einem der Ansprüche 1 bis 4, wobei das Selen in der Form von Natriumselenat, Natrimhydrogenselenit oder Natriumselenit, vorzugsweise von Natriumselenit vorliegt.

6. Verbindung nach einem der Ansprüche 1 bis 5, **dadurch gekennzeichnet, dass** die Carotinoide aus der Gruppe gewählt werden, die das ß-Karotin, das Lycopin, das Lutein, das Asthazanthin, das Zeazanthin und das Canthaxanthin, vorzugsweise das β-Karotin umfassen.

7. Verbindung nach einem der Ansprüche 1 bis 6, **dadurch gekennzeichnet, dass** sie mindestens Vitamin C, Vitamin D, Vitamin E und mindestens ein Vitamin der Gruppe B enthält, ausgewählt aus der Gruppe, die die Vitamine B1, B2, B3, B5, B6, B8, B9 und B12, umfasst, vorzugsweise umfasst sie die Vitamine B1, B2, B3, B5, B6, B8, B9, B12, C, D und E.

8. Verbindung nach Anspruch 7, **dadurch gekennzeichnet, dass**:
(i) das Vitamin B1 in der Form von Thiaminchlorhydrat oder Thiaminmonohydrat, vorzugsweise in der Form von Thiaminchlorhydrat vorliegt;
(ii) das Vitamin B2 in der Form von Riboflavin oder von Riboflavin-5'-Natriumphosphat, vorzugsweise in der Form von Riboflavin vorliegt;
(iii) das Vitamin B3 in der Form von Nikotinsäure oder von Nicotinamid, vorzugsweise in der Form von Nicotinamid vorliegt;
(iv) das Vitamin B5 in der Form von Calcium-D-Pantothenat, Natrium-D-pantothenat oder von Dexpantotenol, vorzugsweise in der Form von Calcium-D-Pantothenat vorliegt;
(v) das Vitamin B6 in der Form von Pyridoxinhydrochlorid, Pyridoxin-5'-Phosphat oder Pyridoxindipalmitat, vorzugsweise in der Form von Pyridoxinhydrochlorid vorliegt;
(vi) das Vitamin B8 in der Form von D-Biotin vorliegt;
(vii) das Vitamin B9 in der Form von Pteroylmonoglutaminsäure vorliegt;
(viii) das Vitamin B12 in der Form von Cyanocobalamin oder Hydroxocobalamin, vorzugsweise in der Form von Cyanocobalamin vorliegt;
(ix) das Vitamin C in der Form von L-Ascorbinsäure, Natrium-L-Ascorbat, Calcium-L-Ascorbat, Kalium-L-Aascorbat, L-Ascorbyl-6-Palmitat oder eines pflanzlichen Extrakts, der reich an Vitamin C ist, wie z.B. eines Acerola-Extrakts, vorzugsweise in der Form eines pflanzlichen Extrakts, der reich an Vitamin C ist, vorliegt;
(x) das Vitamin D in der Form von Cholecalciferol (D₃) oder Ergocalciferol (D₂), vorzugsweise in der Form von Cholecalciferol (D₃) vorliegt;
(xi) das Vitamin E in der Form von D-α-Tocopherol, DL-α-Tocopherol, D-α-Tocopherol-Acetat, DL-α-Tocopherol-Acetat oder D-α-Tocopherol-Sukzinat, vorzugsweise in der Form von DL-α-Tocopherol vorliegt.

9. Verbindung nach einem der Ansprüche 1 bis 8, **dadurch gekennzeichnet, dass** sie u.a. einen Algenextrakt umfasst, der dazu in der Lage ist, die Wirkungen der HSP zu erhöhen, vorzugsweise ein Extakt aus *Porphyra umbilicalis.*

10. Verbindung nach einem der Ansprüche 1 bis 9, **dadurch gekennzeichnet, dass** sie u.a. Plyphenole umfasst, wobei diese Polyphenole in der Form eines Gesamtextraktes aus Trauben, Kakao, Apfel, grünem Tee, Rotwein oder roten Früchten wie z.B. Erdbeeren, Heidelbeeren oder Moosbeeren vorhanden sind, vorzugsweise sind die Polyphenole in der Form eines Gesamtextraktes von Trauben vorhanden.

11. Verbindung nach einem der vorhergehenden Ansprüche, **dadurch gekennzeichnet, dass** die Verbindung, wenn sie für eine tägliche Verabreichung vorgesehen ist, Folgendes umfasst:
(i) natürliche Proteine, reich an Aminosäuren mit verzweigten Ketten, ausgewählt aus der Gruppe umfassend Weizenproteine, Reisproteine, Erbsenproteine, die Lupinenproteine, Sojaproteine und Caseinproteine in einer Menge, die von 5 g bis 35 g, vorzugsweise von 10 g bis 15 g reicht;
(ii) Molkeproteine in einer Menge, die von 8 g bis 20 g, vorzugsweise von 10 g bis 15 g reicht;
(iii) Proteine reich an Arginin und Glutamin in einer Menge, die von 3 g bis 15 g, vorzugsweise von 5 g bis 9 g reicht;
(iv) Ginsenoside in einer Menge, die von 1 mg bis 10 mg, vorzugsweise von 2 mg bis 4 mg reicht;
(v) Zink in einer Menge, die von 10 mg bis 20 mg reicht, vorzugsweise von 13 mg bis 17 mg;
(vi) Selen in einer Menge, die von 10 g bis 500 g, vorzugsweise von 25 µg bis 100 µg reicht;
(vii) Vitamin B1 in einer Menge, die von 0,5 mg bis 5 mg, vorzugsweise von 1 mg bis 2 mg reicht;
(viii) Vitamin B2 in einer Menge, die von 0,5 mg bis 3 mg, vorzugsweise von 1 mg bis 2 mg reicht;
(ix) Vitamin B3 in einer Menge, die von 5 mg bis 30 mg, vorzugsweise von 15 mg bis 20 mg reicht;
(x) Vitamin B5 in einer Menge, die von 1 mg bis 10 mg, vorzugsweise von 5 mg bis 8 mg reicht;
(xi) Vitamin B6 in einer Menge, die von 0,5 mg bis 5 mg, vorzugsweise von 1 mg bis 3 mg reicht;
(xii) Vitamin B8 in einer Menge, die von 0,1 mg bis 1 mg, vorzugsweise von 0,1 mg bis 0,3 mg reicht;
(xiii) Vitamin B9 in einer Menge, die von 0,1 mg bis 0,5 mg, vorzugsweise von 0,15 mg bis 0,3 mg reicht;
(xiv) Vitamin B12 in einer Menge, die von 0,1 µg bis 3 µg, vorzugsweise von 0,5 µg bis 1,5 µg reicht;
(xv) Vitamin C in einer Menge, die von 40 mg bis 100 mg, vorzugsweise von 50 mg bis 80 mg reicht;
(xvi) Vitamin D in einer Menge, die von 1 µg bis 20 µg, vorzugsweise von 5 µg bis 20 µg reicht;
(xvii) Vitamin E in einer Menge, die von 1 mg bis 25 mg, vorzugsweise von 8 mg bis 12 mg reicht;
(xviii) Carotinoide in einer Menge, die von 1 mg bis 20 mg, vorzugsweise von 4,5 mg bis 20 mg reicht;
(xix) ein Algenextrakt in einer Menge, die von 1 mg bis 750 mg, vorzugsweise von 1 mg bis 500 mg reicht; und
(xx) Polyphenole in einer Menge, die von 50 mg bis 500 mg, vorzugsweise von 80 mg bis 150 mg reicht;

12. Verbindung nach einem der vorangehenden Ansprüche, **dadurch gekennzeichnet, dass** sie mindestens Folgendes umfasst:
(i) Molkeproteine, vorzugsweise 11,7 g,
(ii) Erbsenproteine, vorzugsweise 7 g,
(iii) Ginsenoside in der Form von Ginsengpuder, vorzugsweise 2,9 mg Ginsenoside in der Form von 29 mg Ginsengpuder,
(iv) Zink, vorzugsweise 15 mg,
(v) Selen, vorzugsweise 60 µg,
(vi) Vitamin B1, vorzugsweise 1,4 mg,
(vii) Vitamin B2, vorzugsweise 1,6 mg.
(viii) Vitamin B3, vorzugsweise 18 mg.
(ix) Vitamin B5, vorzugsweise 6 mg,
(x) Vitamin B6, vorzugsweise 2 mg,
(xi) Vitamin B8, vorzugsweise 0,15 mg,
(xii) Vitamin B9, vorzugsweise 0,20 mg,
(xiii) Vitamin B12, vorzugsweise 1 µg,
(xiv) Vitamin D, vorzugsweise 5 µg,
(xv) Vitamin C, vorzugsweise 60 mg,
(xvi) Vitamin E, vorzugsweise 10 mg,
(xvii) HSP-Porphyral als Algenextrakt, vorzugsweise 100 mg,
(xviii) Polyphenole in der Form von Traubenpulver, vorzugsweise 100 mg Polyphenole in der Form von 110 mg Traubenpulver, und
(xix) ß-Karotin, vorzugsweise 4,8 mg.

13. Verbindung nach einem der vorangehenden Ansprüche, **dadurch gekennzeichnet, dass** sie außerdem Folgendes umfasst:
(i) Methionin, vorzugsweise 58 mg,
(ii) Magnesium, vorzugsweise 150 mg, und / oder
(iii) Mangan, vorzugsweise 1,75 mg.

14. Verwendung einer Zusammensetzung nach einem der Ansprüche 1 bis 13 für die Zubreitung einer Zusammensetzung, die für die Vorbeugung und / oder Behandlung des Schwächesyndroms bei älteren Personen, des Muskelschwunds, zur Vorbeugung der Schwächung des Immunsystems, zur Vorbeugung der Alterung des Immunsystems, zu Vorbeugung der Schwächung der Zell-Immunantworten, zur Vorbeugung und / oder Reduzierung der Anzahl von Infektionsereignissen, zur Widerherstellung eines oxidationshemmenden Zustandes bei älteren Personen, zur Vorbeugung und / oder Behandlung der posttraumatischen Folgen (früher Post-Hospitalisierung aufgrund von Frakturen), zur Behandlung von Beschwerden bei der Aufrechterhaltung der Haltung und des Ganges, zur Vorbeugung des protein-energetischen Defizits bei älteren Personen, zum Kampf gegen den Verlust der Muskelmasse und der Verringerung der Muskelkraft, zur Vorsorge des Zustandes der Verwundbarkeit und der Schwäche bei älteren Personen.
